# EUROPEAN PATENT SPECIFICATION

(11) **EP 3 280 995 B1**
(45) Date of publication and mention of the grant of the patent: **10.04.2019**
(21) Application number: 16719246.7
(22) Date of filing: 11.04.2016
(51) Int. Cl.: G01N 21/03, G01N 21/25, G01N 21/64, G01N 33/53, B01L 3/00

(54) **ANALYSIS SYSTEM WITH SPACING MEANS**
ANALYSESYSTEM MIT ABSTANDSMITTEL
SYSTÈME D'ANALYSE MUNI D'UN DISPOSITIF D'ESPACEMENT

(30) Priority: 10.04.2015 EP 15163221
(43) Date of publication of application: 14.02.2018
(73) Proprietor: MyCartis NV, 9052 Zwijnaarde (BE)
(72) Inventor: MÜLLER, Robin, 1180 Tartegnin (CH); DE GIL, José, 1024 Ecublens (CH); TORNAY, Raphaël, 1893 Illarsaz (CH); GAILLARD, Matthieu, 1004 Lausanne (CH)
(74) Representative: Gevers Patents
(86) International application number: PCT/EP2016/057931
(87) International publication number: WO 2016/162566

(56) References cited:
- US-A- 5 639 428
- US-A1- 2012 040 470
- US-A1- 2012 156 112
- US-A1- 2013 114 076
- US-A1- 2014 286 826
- US-A1- 2014 349 381
- US-B1- 6 552 784

## Description

The present invention relates to an analysis system comprising a cartridge and an instrument being designed for operating the cartridge and use thereof. The present invention also relates to spacing means and to the instrument for carrying out the analysis system.

Over the past few years, a forward thinking field of medicine focuses on personalized medicine to provide new levels of proteomics and genomics information and paves the way for new and tailored medical services. Personalized medicine aims at predicting, diagnosing or monitoring disease(s) at the level of individual contrary to the "one size fits all" approach. Generally, personalized medicine involves detection assays designed for detecting and quantifying one or more target biomarker(s) comprised in a sample from a patient.

When running a detection assay, the sample is usually comprised in an assay chamber of a cartridge which is processed by a detection device. The detection device usually comprises an observation window coupled to an optical system designed for the optical readout of the content of the assay chamber. In this respect, the assay chamber is conveniently placed opposite the observation window. When one or more target biomarker(s) is detected in the sample, one or more signal(s) is emitted from the assay chamber through the observation window toward the optical system of the detection device.

Typically, the detection of one or more target biomarker(s) is based on biochemical interactions between capture molecules and one or more target biomarker(s) allowing emission of one or more signal(s) which is detected by the optical system. For instance, the optical system can detect a fluorescent signal emitted when an antibody recognizes a target protein.

However, the applicant noticed that when the cartridge is processed with such a detection device, optical effects creating shadows on the observation window frequently occur. Said shadows significantly decrease the transmission of the signal through the observation window and the homogeneity of said signal so that the data provided by the detection assay are degraded by these optical effects.

Another example is disclosed in D1=US2013114076 and describes a device and an examination system providing means for optical examinations of a sample.

Technical features designed to avoid measurement errors are also described in the prior art D2=US6552784 which relates to a disposable optical cuvette device for spectrophotometric measurement of liquid samples.

Therefore, the existing detection device fails to provide reliable and accurate data when it comes to detecting and quantifying targets biomarkers.

The present invention aims to remedy all or part of the disadvantages mentioned above.

The present invention fulfills these objectives by providing an analysis system according to appended claim 1.

According to an embodiment of the present invention, said spacing means comprise a shim.

Furthermore, an instrument is used in the analysis system according to the present invention.

The analysis system according to the present invention is used for detecting at least a target component.

Thus, the present invention solves the problem by providing an analysis system further comprising spacing means to ensure a gap between a foil of a cartridge and a detection portion of an instrument that operates said cartridge.

The applicant found out that the optical effects observed on the detection portion of the instrument according to the prior art are at least partially caused by the contact between the foil sealing the cartridge and the detection portion of the instrument. When the instrument operates the cartridge and the chamber is pressurised, the foil protrudes toward the detection portion and contacts said detection portion. Due to friction forces, the foil does not form a uniform layer but tends to generate the aforementioned optical effects, i.e. the shadows also called ripples.

Therefore, the applicant discovered that when the chamber is pressurized, the spacing means of the analysis system according to the present invention ensures a gap between the foil and the detection portion. Thus, the spacing means prevent any contact between the foil and the detection portion to avoid the optical effects observed in the prior art.

Moreover, the applicant discovered that with the analysis system of the prior art, when the chamber is pressurized and the foil contacts the detection portion, the number of transmission media crossed by a signal from the chamber to the instrument varies between a first point and a second point of the detection portion. Hence, with the analysis system according to the prior art:
- the signal will cross "n" transmission media at the first point of the detection portion where the foil does contact the detection portion;
- whereas at the second point where the foil does not contact the detection portion, there is a space between the foil and the detection portion so that the signal will cross an additional transmission media, i.e. the space, so that the transmission medium cross by the signal at said second point will be "n+1".

Contrary to the analysis system according to the prior art, in the present invention when the chamber is pressurized, the spacing means prevent the contact between the foil and the detection portion so that the number of transmission media is constant at any point of the detection portion. Thus, the spacing means of the analysis system according to the present invention allows that the number of transmission media crossed by the signal from the chamber to the instrument is constant at any point of the detection portion.

Additionally, it has also been discovered that, with the analysis system of the prior art, when the foil contacts the detection portion on a contact point of the instrument creating the aforementioned optical effects, the transmission of the signal that crossed the detection portion at said contact point decreased (typically by 20%). In the present invention, the spacing means prevent the contact point and hence the decrease of the transmission of signal related thereof. Thus, the spacing means of the analysis system according to the present invention allows improving the sensitivity of the analysis system by detecting signal that would not have been detected by analysis system according to the prior art. In this respect, the spacing means also permit to improve the efficiency of the detection by preventing any contact point and hence the decrease in the transmission of the signal related thereof. The quantification is also improved.

According to an embodiment, the spacing means are separable from the analysis system. Thus, the spacing means can be installed on instrument of the prior art.

The electromagnetic signals are electromagnetic signals of which the wavelengths are comprised in a range between deep ultraviolet and deep infrared.

The electromagnetic signals are emitted from the chamber.

In an embodiment, the instrument further comprises a support comprising a recess shaped to accommodate the analysis window and the spacing means, said recess further comprising an opening opposite said analysis window.

The chamber is designed for being pressurized up to 7 bars.

In an embodiment, the thickness of the shim is adapted to the foil and to the foil dimensions in order to be greater than the maximum deformation of the foil when the chamber is pressurized.

In an embodiment, the instrument further comprises heating element, said heating element being capable of transferring heat to the spacing means. Therefore, when the instrument operates the cartridge, a first part of the cartridge contacting the instrument can be at the same temperature than a second part of the cartridge contacting the spacing means. Thus, the variation of temperature between the first part of the cartridge and the second part of the cartridge is minimized.

According to an embodiment, when the cartridge is docked to the instrument via the docking means and the chamber is pressurized, the distance (d) between said detection portion and said foil is comprised between about 1 micrometer and about 250 micrometer, preferably between about 5 micrometer and about 100 micrometer, more preferably between about 10 micrometer and about 50 micrometer.

In an embodiment, the instrument comprises the spacing means.

In another embodiment, the spacing means cooperate with the analysis window.

According to a technical feature, the spacing means are designed for being placed opposite the analysis window.

According to an embodiment, the shim has a thermal conductivity between about 300 W/m K and about 1000 W/m K at 20°C. Thus, when the shim is heated, the heat is transferred to the part of the cartridge contacting the shim.

The spacing means, more particularly the shim, can be made or comprise(s) any material. In one embodiment, the shim is made of or comprises metal, more particularly the shim is made of or comprises copper.

In an embodiment, the shim further comprises reversible fastening means for fastening the shim to the instrument. Thus, when the shim is fastened to the fastening means, the shim is integral with the instrument. Furthermore, when the cartridge is docked to the instrument via the docking means and the shim is fastened to the instrument via the fastening means, the cartridge is integral with the shim. Moreover, the fastening means ensure the positioning of the shim with respect to the analysis window to maintain the gap between the detection portion and the foil of the cartridge when the cartridge is docked to the instrument.

In one embodiment, the fastening means comprises a first part and a second part, the first part comprising a tab extending from the perimeter of the shim, the second part comprising a hollow shaped in the instrument, so that when the tab is received in the hollow, the shim is fastened to the instrument.

According to an embodiment, the shim is designed for being placed on the analysis window.

In an embodiment, when the shim is placed opposite the analysis window, the shim and the support are coplanar meaning that the shim comes up with the support. In this embodiment, the support comprising the analysis window and the shim define a plan surface on the support. Thus, when the instrument operates the cartridge, the cartridge lays flat on the plan surface of support of the instrument. Therefore, if the cartridge is heated by the instrument, for instance via the heating element, the heat loss between the instrument and the cartridge is minimized.

In another embodiment, when the shim is placed opposite the analysis window, said shim defines a surface on the analysis window that matches with the detection portion of the analysis window.

The spacing means, more particularly the shim, can be of any shape. According to an embodiment, the shim is U shaped.

In an embodiment, the shim has a thickness between about 50 micrometer and about 250 micrometer, preferably between about 100 micrometer and about 200 micrometer, more preferably between about 130 micrometer and about 170 micrometer.

The present invention is further illustrated by the following detailed description set forth in view of the appended drawings, which represent an exemplary and explanatory embodiment of an analysis system according to the present invention:
Figure 1 illustrates a top view of an analysis system according to the present invention;
Figure 2 illustrates a cross section view of the analysis window of the instrument of the analysis system when said instrument is operating the cartridge.

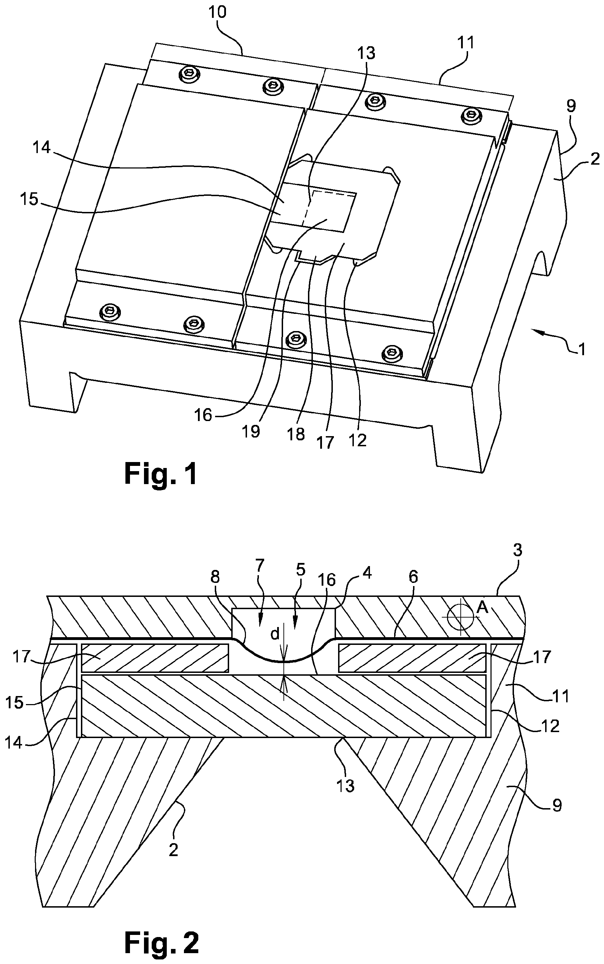

An analysis system 1 according to the present invention, partially illustrated in figures 1 and 2, comprises an instrument 2 and a disposable cartridge 3 shown in figures 1 and 2. The cartridge 3 and the instrument 2 are separable. The analysis system 1 according to the present invention can be used to perform an analysis of a liquid solution comprising a sample from a patient. To that end, the liquid solution is introduced in the disposable cartridge 3. Typically, the analysis aims at detecting and quantifying a biomarker or biomarkers panel from the sample in order to diagnose a disease, for instance cardiovascular disease. In this respect, the sample can be whole blood or a fractional component thereof such as plasma or serum.

The cartridge 3 comprises a chamber 4 formed by a cavity 5 in a portion 6 of the cartridge 2. In the embodiment shown in figure 2, the chamber 4 is formed by a channel 7 extending along the axis A of the cartridge 3. The channel 7 is connected to pressurising means (not shown in figures) designed for pressurizing the chamber 4 thereby permitting a flow of the liquid solution through the channel 7. The chamber 4 further comprises microparticles (not shown in figures) functionalized to emit electromagnetic signals toward the instrument when the targeted biomarker is detected in the sample. For instance, the surface of said microparticles can be grafted with antibodies designed for recognizing the biomarker and providing a fluorescent signal thereupon. The chamber 4 is sealed by a foil 8 extending along the portion 6 of the cartridge 3. The foil 8 is made of or comprises a deformable material allowing adjusting the volume of the chamber 4 depending on the pressure applied to the chamber 4 by the pressure means while maintaining the chamber 4 sealed. In the present embodiment, the foil 8 comprises Cyclic Olefin Polymer (COC). As illustrated in figure 2, the foil 8 protrudes when the chamber 4 is pressurized.

The instrument 2 of the embodiment presented in figures 1 and 2 comprises a support 9 designed for being placed opposite the cartridge 3. In the embodiment presented in figures 1 and 2, the support 9 has a rectangular shaped and further comprises heating element A 10 and heating element B 11 screwed on the support 9. The instrument further comprises a recess 12, said recess further comprising an opening 13. The recess 12 is shaped to accommodate at least an analysis window 14, said analysis window 14 being placed opposite the opening 13. The analysis window 14 is transparent to electromagnetic signals and further comprises a detection portion 16 designed for being crossed by the signal emitted from the chamber 4. In the embodiment presented in figures 1 and 2, the analysis window 14 is a sapphire window 15 and the detection portion 16 is designed for being crossed by the fluorescent signal emitted by the microparticle comprised in the chamber 4 upon detection of the targeted biomarkers. The instrument further comprises docking means for docking the cartridge 3 to the instrument 2. For instance, the docking means comprise a slot and a stopper (not showed in figures) so that when the cartridge 3 is docked in the docking means, the chamber 4 is placed opposite the detection portion 16. Thus, when the chamber 4 is pressurized during the assay, the foil 8 of the chamber 4 protrudes toward said detection portion 16.

The analysis system 1 further comprises spacing means to ensure that, when the cartridge 3 is docked to the instrument 2 via the docking means and the chamber 4 is pressurized, said spacing means ensure a gap between the foil 8 and the detection portion 16. For instance, in the embodiment shown in figure 1, the spacing means ensure that the gap, i.e. the distance (d), between the foil 8 and the detection portion 16 is at least 10 micrometer. In the embodiment shown in figures 1 and 2, the spacing means comprise a shim 17, said shim 17 being shaped to surround at least partially the detection portion 16. In the present case, the shim 17 is 150 micrometer thick. In the embodiment illustrated in figures 1 and 2, the shim 17 is U shaped. When the shim 17 is placed on the analysis window 14, said U shaped shim 17 defines a surface on the analysis window that matches with the detection portion 16 of the analysis window 14. The shim 17 is made of copper because copper has an appropriate thermal conductivity (385 W/(m K) at 20°C) to ensure the heat transfer between the heating element B 11 and the cartridge 3. In the embodiment illustrated on figure 1, the shim 17 further comprises a tab 18 extending from the perimeter of the shim 17, said tab 18 being designed for being received in a hollow 19 of the heating element B 11 to fasten the shim 17 to the heating element B 11.

Other embodiments of the invention will be apparent to those skilled in the art from consideration of the specification and practice of the invention disclosed herein. It is intended that the specification and examples be considered as exemplary only, with the true scope of the invention being indicated by the following claims.

## Claims

1. An analysis system (1) comprising a cartridge (3) and an instrument (2) being designed for operating the cartridge (3),
the cartridge (3) comprising a chamber (4) formed by a cavity (5) in a portion (6) of the cartridge (3), said chamber (4) being sealed by a foil (8) extending along said portion (6), said foil (8) being capable of protruding when the chamber (4) is pressurized up to 7 bars,
the instrument (2) comprising an analysis window (14) being transparent to electromagnetic signals of which the wavelengths are comprised in a range between deep ultraviolet and deep infrared, said analysis window (14) comprising a detection portion (16) designed for being crossed by a signal emitted from the chamber (4) toward the instrument (2) when the chamber (4) is placed opposite the detection portion (16),
the instrument (2) further comprising docking means for docking the cartridge (3) on the instrument (2) in order to place the chamber (4) opposite the detection portion (16) so that when the chamber (4) is pressurized, the foil (8) protrudes toward the detection portion (16),
the analysis system (1) being **characterized in that** it further comprises spacing means to ensure that, when the cartridge (3) is docked on the instrument (2) via the docking means and the chamber (4) is pressurized, said spacing means ensure a gap between the foil (8) and the detection portion (16).

2. The analysis system (1) according to claim 1, wherein the spacing means are separable from the analysis system (1).

3. The analysis system (1) according to claims 1 or 2, wherein the instrument (2) further comprises a support (9) comprising a recess (12) shaped to accommodate the analysis window (14) and the spacing means, said recess (12) further comprising an opening (13) opposite said analysis window (14).

4. The analysis system (1) according to any one of claims 1 to 3, wherein the instrument (2) further comprises heating element (10,11), said heating element (10, 11) being capable of transferring heat to the spacing means.

5. The analysis system (1) according to any one of claims 1 to 4, wherein when the cartridge (3) is docked to the instrument (2) via the docking means and the chamber (4) is pressurized, the distance (d) between said detection portion (16) and said foil (8) is comprised between about 1 micrometer and about 250 micrometer, preferably between about 5 micrometer and about 100 micrometer, more preferably between about 10 micrometer and about 50 micrometer.

6. Use of an analysis system (1) according to anyone of claims 1 to 5 for detecting at least a target component.

## Patentansprüche

1. Ein Analysesystem (1), welches eine Kartusche (3) und ein Instrument (2) umfasst, welches zum Bedienen der Kartusche (3) entworfen ist,
wobei die Kartusche (3) eine Kammer (4) umfasst, gebildet durch einen Hohlraum (5) in einem Abschnitt (6) der Kartusche (3), wobei die erwähnte Kammer (4) durch eine Folie (8) versiegelt ist, welche sich entlang des erwähnten Abschnitts (6) ausdehnt, wobei die erwähnte Folie (8) in der Lage ist, hervorzuragen, wenn die Kammer (4) mit einem Druck von bis zu 7 bar beaufschlagt wird,
wobei das Instrument (2) ein Analysefenster (14) umfasst, welches für elektromagnetische Signale durchlässig ist, deren Wellenlängen in einem Bereich zwischen tief ultraviolett und tief infrarot liegen, wobei das erwähnte Analysefenster (14) einen Erkennungsabschnitt (16) umfasst, entworfen, um durch ein Signal durchquert zu werden, welches von der Kammer (4) hin zum Instrument (2) gesendet wird, wenn die Kammer (4) gegenüber dem Erkennungsabschnitt (16) positioniert wird,
wobei das Instrument (2) ferner Kopplungsmittel zum Koppeln der Kartusche (3) an das Instrument (2) umfasst, um die Kammer (4) gegenüber dem Erkennungsabschnitt (16) zu positionieren, sodass die Folie (8), wenn die Kammer (4) mit Druck beaufschlagt wird, hin zum Erkennungsabschnitt (16) ragt,
wobei das Analysesystem (1) **dadurch gekennzeichnet ist, dass** es ferner Abstandsmittel umfasst, um sicherzustellen, dass, wenn die Kartusche (3) über die Kopplungsmittel an das Instrument (2) gekoppelt ist und die Kammer (4) mit Druck beaufschlagt wird, die Abstandsmittel einen Spalt zwischen der Folie (8) und dem Erkennungsabschnitt (16) gewährleisten.

2. Das Analysesystem (1) nach Anspruch 1, wobei die Abstandsmittel vom Analysesystem (1) abnehmbar sind.

3. Das Analysesystem (1) nach den Ansprüchen 1 oder 2, wobei das Instrument (2) ferner einen Träger (9) mit einer Vertiefung (12) umfasst, geformt, um das Analysefenster (14) und die Abstandsmittel aufzunehmen, wobei die erwähnte Vertiefung (12) ferner eine Öffnung (13) gegenüber dem erwähnten Analysefenster (14) umfasst.

4. Das Analysesystem (1) nach irgendeinem der Ansprüche 1 bis 3, wobei das Instrument (2) ferner ein Wärmeelement (10, 11) umfasst, wobei das erwähnte Wärmeelement (10, 11) in der Lage ist, Wärme auf die Abstandsmittel zu übertragen.

5. Das Analysesystem (1) nach irgendeinem der Ansprüche 1 bis 4, wobei, wenn die Kartusche (3) über die Kopplungsmittel an das Instrument (2) gekoppelt ist und die Kammer (4) mit Druck beaufschlagt ist, der Abstand (d) zwischen dem erwähnten Erkennungsabschnitt (16) und der erwähnten Folie (8) zwischen ungefähr 1 Mikrometer und ungefähr 250 Mikrometer liegt, bevorzugt zwischen ungefähr 5 Mikrometer und ungefähr 100 Mikrometer, noch besser zwischen ungefähr 10 Mikrometer und ungefähr 50 Mikrometer.

6. Verwendung eines Analysesystems (1) nach irgendeinem der Ansprüche 1 bis 5 zum Erkennen von zumindest einer Zielkomponente.

## Revendications

1. Un système d'analyse (1) comprenant une cartouche (3) et un instrument (2) conçu pour mettre en oeuvre la cartouche (3),
la cartouche (3) comprenant une chambre (4) formée par une cavité (5) dans une portion (6) de la cartouche (3), ladite chambre (4) étant scellée par une feuille (8) s'étendant le long de ladite portion (6), ladite feuille (8) étant susceptible de faire saillie quand la chambre (4) est pressurisée jusqu'à 7 bars,
l'instrument (2) comprenant une fenêtre d'analyse (14) qui est transparente à des signaux électromagnétiques dont les longueurs d'onde sont comprises dans une plage entre l'ultraviolet profond et l'infrarouge profond, ladite fenêtre d'analyse (14) comprenant une portion de détection (16) conçue pour être traversée par un signal émis en provenance de la chambre (4) vers l'instrument (2) lorsque la chambre (4) est placée en face de la portion de détection (16),
l'instrument (2) comprenant en outre un moyen d'arrimage pour arrimer la cartouche (3) sur l'instrument (2) afin de placer la chambre (4) en face de la portion de détection (16) de sorte que lorsque la chambre (4) est pressurisée, la feuille (8) fait saillie vers la portion de détection (16),
le système d'analyse (1) étant **caractérisé en ce qu'**il comprend en outre un moyen d'espacement pour assurer que, lorsque la cartouche (3) est arrimée sur l'instrument (2) via le moyen d'arrimage et que la chambre (4) est pressurisée, ledit moyen d'espacement assure un espacement entre la feuille (8) et la portion de détection (16).

2. Le système d'analyse (1) selon la revendication 1, dans lequel le moyen d'espacement peut être séparé du système d'analyse (1).

3. Le système d'analyse (1) selon les revendications 1 ou 2, dans lequel l'instrument (2) comprend en outre un support (9) comprenant une partie en retrait (12) formée pour loger la fenêtre d'analyse (14) et le moyen d'espacement, ladite partie en retrait (12) comprenant en outre une ouverture (13) en face de ladite fenêtre d'analyse (14).

4. Le système d'analyse (1) selon l'une quelconque des revendications 1 à 3, dans lequel l'instrument (2) comprend en outre un élément chauffant (10, 11), ledit élément chauffant (10, 11) étant susceptible de transférer de la chaleur au moyen d'espacement.

5. Le système d'analyse (1) selon l'une quelconque des revendications 1 à 4, dans lequel lorsque la cartouche (3) est arrimée sur l'instrument (2) via le moyen d'arrimage et que la chambre (4) est pressurisée, la distance (d) entre ladite portion de détection (16) et ladite feuille (8) est comprise entre environ 1 micromètre et environ 250 micromètres, de préférence entre environ 5 micromètres et environ 100 micromètres, de manière plus préférée entre environ 10 micromètres et environ 50 micromètres.

6. Utilisation d'un système d'analyse (1) selon l'une quelconque des revendications 1 à 5 pour détecter au moins un composant cible.
